# EUROPEAN PATENT APPLICATION

(11) **EP 4 282 382 A1**
(43) Date of publication of application: **29.11.2023**
(21) Application number: 22863096.8
(22) Date of filing: 15.08.2022
(51) Int. Cl.: A61F 2/24, A61M 25/08

(54) **IMPLANT DELIVERY HANDLE, IMPLANT SYSTEM, IMPLANT DELIVERY SYSTEM AND USE METHOD THEREFOR**

(30) Priority: 23.11.2021 CN 202111397065
(71) Applicant: Shanghai Trulive Medtech Co., Ltd, Shanghai 201206 (CN); Jiangsu Trulive Medtech Co., Ltd, Rudong County Nantong Jiangsu 226400 (CN)
(72) Inventor: ZHANG, Wei, Shanghai 201206 (CN); XU, Haoran, Shanghai 201206 (CN); WEI, Yongqiang, Shanghai 201206 (CN); ZHAO, Jing, Shanghai 201206 (CN)
(74) Representative: Nordmeyer, Philipp Werner
(86) International application number: PCT/CN2022/112376
(87) International publication number: WO 2023/029956

(57) **Abstract**

An implant delivery handle, an implant system, a delivery system and a method of use thereof are disclosed. Transmission systems in the implant delivery handle each include a lead screw, an inner tube coupling member, an outer gear and an inner gear. The outer gear rotatably engages the inner gear and is configured to drive rotation of the inner gear. The lead screw is coupled to a first inner tube (11) or a second inner tube (12) through the inner tube coupling member. The lead screw also engages the inner gear and rotates therewith to cause axial movement of the first inner tube (11) or the second inner tube (12). A transmission ratio of the outer gear to the inner gear is greater than 1. In this way, the outer and inner gears make up a planetary gearset, which functions together with the lead screw to enable faster power transmission in the implant delivery system, which results in a reduction in the time required for a surgical procedure using the device and hence in the time over which the device stays in the patient's body, lowering the risk of accidents in the surgical procedure.

## Description

### TECHNICAL FIELD

The present invention relates to the field of medical devices and, in particular, an implant delivery handle, an implant system, a delivery system and a method of use thereof.

### BACKGROUND

The heart has four chambers: the left atrium and ventricle on the left side of the heart; and the right atrium and ventricle on the right side of the heart. It also has the ventricular inflow tracts between the atria and ventricles, the left ventricular outflow tract consisting of the left ventricle and aorta, and the right ventricular outflow tract consisting of the right ventricle and pulmonary artery. In the ventricular inflow and outflow tracts, there are "one-way valves" which ensure correct blood flow in the heart. Defects in any of these valves can induce hemodynamic changes and functional abnormalities in the heart, which are known as valvular heart disease.

With the development of social economy and the aging of population, the incidence of valvular heart disease has increased significantly. Studies have shown that this figure has reached up to 13.3% among those 75 years or older. At present, traditional surgical treatment remains the first choice for patients with severe valvular disease. However, for those with advanced ages, complications in multiple organs, a history of thoracotomy or poor heart functions, the traditional surgical approach is associated with high risk and high mortality or even precludes some patients.

Interventional valve implantation is a brand new minimally invasive valve replacement technique that has been developed abroad in recent years, which involves loading a prosthetic valve in a delivery system and delivering it into a human body through a catheter. The prosthesis can functionally replace the patient's dysfunctional native valve and improve his/her cardiac condition. This technique is able to treat valvular heart disease, without surgically opening the chest or stopping the heartbeat, which may cause significant trauma to the patient.

The structure of the human heart is very complex. In particular, the structure of the mitral valve is more complex even than that of the aortic valve because the mitral annulus has an irregular shape and there are many chordae tendineae in the ventricular chamber, which pose great challenges to the implantation and positioning of a prosthetic valve. For transcatheter valve replacement (including transcatheter aortic valve replacement (TAVI), transcatheter mitral valve replacement (TMVR)), safe and effective operation of the delivery system used is one of the key factors for surgical success. Accordingly, delivery systems for this purpose are required to allow safe and accurate operation and, in particular, to provide desirable power transmission efficiency and sealing performance. The power transmission efficiency of such a delivery system can affect the time required for a surgical procedure using the system, and poor sealing performance of the delivery system, especially of a delivery catheter thereof, may cause blood loss, gas leakage and other problems, which may lead to an increase in risk associated with the surgical procedure.

### SUMMARY OF THE INVENTION

It is an objective of the present invention to provide an implant delivery handle, an implant system, a delivery system and a method of use thereof. The delivery system has higher power transmission efficiency, which can reduce the time required for a surgical procedure using it.

It is another objective of the present invention to improve sealing performance of a catheter assembly, thereby reducing gas and liquid leakage and lowering risk associated with a surgical procedure using the catheter assembly.

To this end, the present invention provides an implant delivery handle including two transmission systems arranged along an axial direction of a first inner tube. A distal one of the transmission systems is coupled to a proximal end of the first inner tube, and a proximal one of the transmission systems is coupled to a proximal end of a second inner tube. The implant delivery handle is wherein

each of the transmission systems includes a lead screw, an inner tube coupling member, an outer gear and an inner gear, the outer gear rotatably engaging the inner gear and configured to drive the inner gear to rotate, the lead screw coupled to the first or second inner tube through the inner tube coupling member, the lead screw also engaging the inner gear and rotating therewith to drive the first or second inner tube to axially move, wherein a transmission ratio of the outer gear to the inner gear is greater than 1.

Optionally, the outer gear may be disposed over, and mesh with, the inner gear.

Optionally, the inner tube coupling member in the distal transmission system may be coupled to an outer wall surface of the first inner tube around its proximal end, disposed over the second inner tube and configured to seal a gap between the proximal end of the first inner tube and the second inner tube.

Additionally, the inner tube coupling member in the proximal transmission system may be coupled to an outer wall surface of the second inner tube around its proximal end, disposed over a guidewire tube in a catheter assembly and configured to seal a gap between the proximal end of the second inner tube and the guidewire tube.

Optionally, the inner tube coupling member in the distal transmission system may include a first seal, a second seal and a third seal, which are disposed over the second inner tube, wherein the proximal end of the first inner tube is snugly secured in a distal end of the first seal, and the second seal is elastically switched between a proximal end of the first seal and a distal end of the third seal and, when compressed by both the first and third seals, fits against the outer wall surface of the second inner tube, thereby sealing the gap between the proximal end of the first inner tube and the second inner tube.

Additionally, the inner tube coupling member in the proximal transmission system may include a fourth seal disposed over the guidewire tube in the catheter assembly, a fifth seal and a sixth seal, wherein the proximal end of the second inner tube is snugly secured in a distal end of the fourth seal, and the fifth seal is elastically switched between a proximal end of the fourth seal and a distal end of the sixth seal and when compressed by both the fourth and sixth seals, fits against an outer wall surface of the guidewire tube, thereby sealing the gap between the proximal end of the second inner tube and the guidewire tube.

Further, the second and fifth seals may each be made of a material including a polymeric material, and the second and fifth seals may each have a Shore A hardness of 35 HA to 55 HA.

Further, the first, second and third seals may be all tubular structures, wherein the second seal is disposed within the proximal end of the first seal, and the distal end of the third seal is disposed within the proximal end of the first seal and configured to act together with the first seal to compress the second seal so that the second seal experiences an inner diameter decrease and fits against the second inner tube.

Additionally, inner diameters of the first, second and third seals may be all greater than or equal to an outer diameter of the second inner tube, wherein the inner diameter of the first seal is greater at its proximal end than at its distal end, wherein an outer diameter of the second seal lies between the inner diameter of the first seal at its distal end and the inner diameter of the first seal at its proximal end, and wherein an outer diameter of the third seal at its distal end lies between the inner diameter of the second seal at its distal end and the inner diameter of the first seal at its proximal end.

Further, the fourth, fifth and sixth seals may be all tubular structures, wherein the fifth seal is disposed within the proximal end of the fourth seal, and the distal end of the sixth seal is disposed within the proximal end of the fourth seal and configured to act together with the fourth seal to compress the fifth seal so that the fifth seal experiences an inner diameter decrease and fits against the guidewire tube.

Additionally, inner diameters of the fourth, fifth and sixth seals may be all greater than or equal to an outer diameter of the guidewire tube, wherein the inner diameter of the fourth seal is greater at its proximal end than at its distal end, wherein an outer diameter of the fifth seal lies between the inner diameter of the fourth seal at its distal end and the inner diameter of the fourth seal at its proximal end, and wherein an outer diameter of the sixth seal at its distal end lies between the inner diameter of the fifth seal at its distal end and the inner diameter of the fourth seal at its proximal end.

Optionally, the lead screw may be arranged coaxially with the inner gear and has an external thread, wherein the inner tube coupling member has an internal thread and threadedly engages with the lead screw, and wherein as a result of threaded rotation of the lead screw, the inner tube coupling member drives axial movement of the first or second inner tube.

In another aspect, the present invention provides an implant system including the implant delivery handle as defined above and a catheter assembly, the catheter assembly including a first inner tube, a second inner tube and a guidewire tube, which nest one within another from the outside inwards, wherein the implant delivery handle drives axial movement of the first and second inner tubes.

In yet another aspect, the present invention provides a delivery system including the implant delivery handle as defined above, a first inner tube, a second inner tube, a guidewire tube, a tapered head and a retaining head, the retaining head disposed at distal ends of the second inner tube and the guidewire tube, the tapered head disposed at a distal end of the first inner tube, wherein an implant is crimped within a distal end portion of the first inner tube between the tapered head and the retaining head, and wherein the implant delivery handle drives axial movement of the first and second inner tubes, thereby achieving loading, delivery and release of the implant.

In a further aspect, the present invention provides a method of use of the delivery system as defined above. The method includes:
rotating the outer gear in a first direction, causing simultaneous rotation of the inner gear with the outer gear, and of the lead screw with the inner gear, which in turn causes the inner tube coupling member to drive axial advancement of the first or second inner tube; and
rotating the outer gear in the direction opposite to the first direction, causing synchronous rotation of the inner gear with the outer gear, and of the lead screw with the inner gear, which in turn causes the inner tube coupling member to drive axially retraction of the first or second inner tube.

Compared with the prior art, the present invention has the benefits as follows:

It provides an implant delivery handle, an implant system, a delivery system and a method of use thereof. Transmission systems in the implant delivery handle each include a lead screw, an inner tube coupling member, an outer gear and an inner gear. The outer gear rotatably engages the inner gear and is configured to drive rotation of the inner gear. The lead screw is coupled to a first inner tube or a second inner tube through the inner tube coupling member. The lead screw also engages the inner gear and rotates therewith to cause axial movement of the first or second inner tube. A transmission ratio of the outer gear to the inner gear is greater than 1. In this way, the outer and inner gears make up a planetary gearset, which functions together with the lead screw to enable faster power transmission in the implant delivery system, which results in a reduction in the time required for a surgical procedure using the device and hence in the time over which the device stays in the patient's body, lowering the risk of accidents in the surgical procedure.

Further, the inner tube coupling member in a distal one of the transmission systems is coupled to an outer wall surface of the first inner tube around its proximal end, disposed over the second inner tube and configured to seal a gap between the proximal end of the first inner tube and the second inner tube. Moreover, the inner tube coupling member in a proximal one of the transmission systems is coupled to an outer wall surface of the second inner tube around its proximal end, disposed over a guidewire tube and configured to seal a gap between the proximal end of the second inner tube and the guidewire tube. In this way, good sealing performance is achieved at the proximal end of the catheter assembly. Furthermore, the present invention has advantages including a simple structure, high safety and low cost.

### BRIEF DESCRIPTION OF THE DRAWINGS

Fig. 1 is a schematic perspective diagram showing the structure of an implant delivery system according to an embodiment of the present invention.
Figs. 2a to 2b are schematic partial views of an implant delivery system according to an embodiment of the present invention.
Fig. 3 is a schematic partial view of an implant delivery handle according to an embodiment of the present invention.
Figs. 4a to 4b are schematic diagrams showing the structure of a first power transmission assembly according to an embodiment of the present invention.
Figs. 5a to 5b are schematic diagrams showing the structure of a first linkage assembly according to an embodiment of the present invention.
Figs. 6a to 6f are schematic diagrams showing the structures of various components in a first linkage assembly according to an embodiment of the present invention.
Figs. 7a to 7c are schematic diagrams showing the structure of a first linkage assembly according to an embodiment of the present invention during a sealing process.

In these figures:
11-first inner tube; 12-second inner tube; 13-guidewire tube; 2-first power transmission assembly; 21-first outer gear; 22-first inner gear; 3-housing; 4-second power transmission assembly; 41-second outer gear; 42-second inner gear; 43-guidewire tube fastener; 5-first linkage assembly; 51-first inner tube coupling member; 511-first portion; 512-first fastener; 513-second portion; 513a-first seal; 513b-second seal; 513c-third seal; 5131-first distal portion; 5132-first transition portion; 5133-first proximal portion; 5134-second proximal portion; 5135-second distal portion; 5136-third proximal portion; 5137-third distal portion; 5138-first trough; 52-first lead screw; 6-second linkage assembly; 61-second inner tube coupling member; 62-second lead screw;
7-implant.

### DETAILED DESCRIPTION

In principle, the present invention provides an implant delivery handle including two transmission systems arranged along an axially direction of a first inner tube. A distal one of the transmission systems is coupled to a proximal end of the first inner tube, and a proximal one of the transmission systems is coupled to a proximal end of a second inner tube.

Each transmission system includes a lead screw, an inner tube coupling member, an outer gear and an inner gear. The outer gear rotatably engages the inner gear and is configured to drive the inner gear to rotate. The lead screw engages the first or second inner tube through the inner tube coupling member. The lead screw also engages the inner gear and rotates therewith to cause axial movement of the first or second inner tube. A transmission ratio of the outer gear to the inner gear is greater than 1.

In another aspect, the present invention provides an implant delivery system including the implant delivery handle as defined above and a catheter assembly. The catheter assembly includes a first inner tube, a second inner tube and a guidewire tube, which nest one within another from the outside inwards. The implant delivery handle drives the first and second inner tubes to axially move.

In yet another aspect, the present invention provides an implant system including the implant delivery handle as defined above, a first inner tube, a second inner tube, a guidewire tube, a tapered head and a retaining head. The retaining head is disposed at distal ends of the second inner tube and the guidewire tube, and the tapered head is disposed at a distal end of the first inner tube. An implant is crimped within a distal end of the first inner tube between the tapered and retaining heads. The implant delivery handle drives the first and second inner tubes to axially move to effect loading, delivery and release of the implant.

In a further aspect, the present invention provides a method of use of the implant delivery system as defined above. The method includes:
rotating the outer gear in a first direction, causing synchronous rotation of the inner gear with the outer gear, and of the lead screw with the inner gear, which in turn causes the inner tube coupling member to drive the first or second inner tube to axially advance; and
rotating the outer gear in the direction opposite to the first direction, causing synchronous rotation of the inner gear with the outer gear, and of the lead screw with the inner gear, which in turn causes the inner tube coupling member to drive the first or second inner tube to axially retract.

The implant delivery handle, the implant system, the delivery system and the method proposed in the present invention will be further described below. The present invention will be described in greater detail below with reference to the accompanying drawings, which present preferred embodiments of the invention. It would be appreciated that those skilled in the art can make changes to the invention disclosed herein while still obtaining the beneficial results thereof. Therefore, the following description shall be construed as being intended to be widely known by those skilled in the art rather than as limiting the invention.

For the sake of clarity, not all features of actual implementations are described. In the following, description and details of well-known functions and structures are omitted to avoid unnecessarily obscuring the invention. It should be appreciated that in the development of any such actual implementation, numerous implementation-specific decisions must be made to achieve specific goals of the developers, such as compliance with system-related and business-related constrains, which may vary from one implementation to another. Moreover, it should be appreciated that such a development effort might be complex and time consuming, but would nevertheless be a routine undertaking for those of ordinary skill in the art.

Objects and features of the present invention will become more apparent upon reading the following more detailed description of particular embodiments thereof reference to the accompanying drawings. Note that the figures are provided in a very simplified form not necessarily drawn to exact scale and for the only purpose of facilitating easy and clear description of the disclosed embodiments. As used herein, the term "or" is generally employed in the sense of "and/or", unless the context clearly dictates otherwise. As used herein, the terms "inner", "outer" and similar terms are merely illustrative and do not represent the only implementation possible. As used herein, the terms "proximal" and "distal" are employed to describe relative orientations, relative positions and directions between components of a medical device or actions thereof, as viewed by a physician operating the device. Without wishing to be limiting, a "proximal end" usually refers to an end closer to the operator, and a "distal end" usually refers to an end closer to the heart of a patient, during normal operation of the medical device.

Further, the first and second power transmission assemblies described herein are substantially of the same structure except for some subtle differences. For example, the first power transmission assembly defines a first through hole configured for insertion of a first inner tube therethrough, while the second power transmission assembly is provided with a guidewire tube fastener for securing a guidewire tube. These differences do not affect actual functioning of the first and second power transmission assemblies. Likewise, the first and second linkage assemblies described herein are substantially of the same structure except for some subtle differences, for example, in axial length of first and second lead screws and dimensions of first and third portions respectively of first and second inner tube coupling members. These differences do not affect actual functioning of the first and second linkage assemblies either. Accordingly, for the sake of brevity and clarity, only the first power transmission assembly in the following embodiments is illustrated in detail in the annexed figures, while both the first and second power transmission assemblies are described in greater detail in the following description of the particular embodiments disclosed herein.

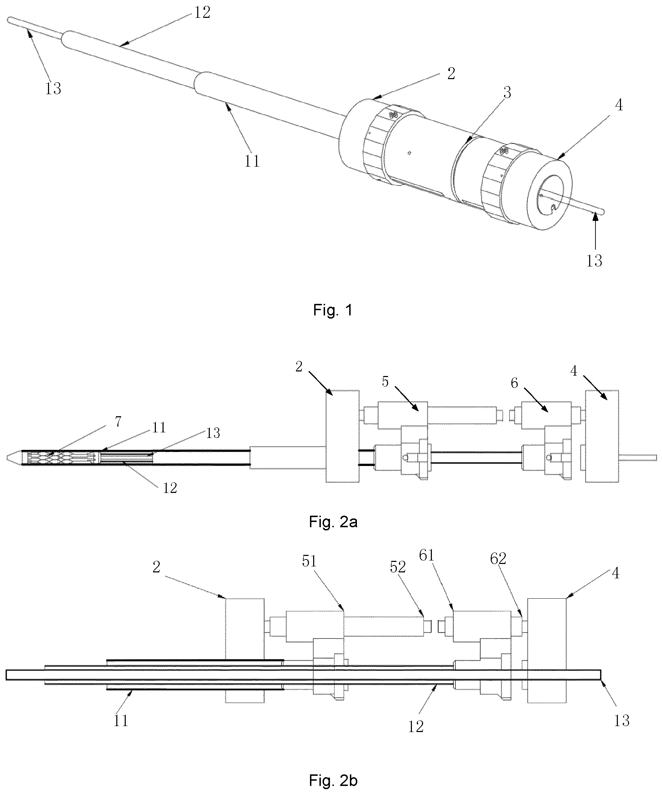

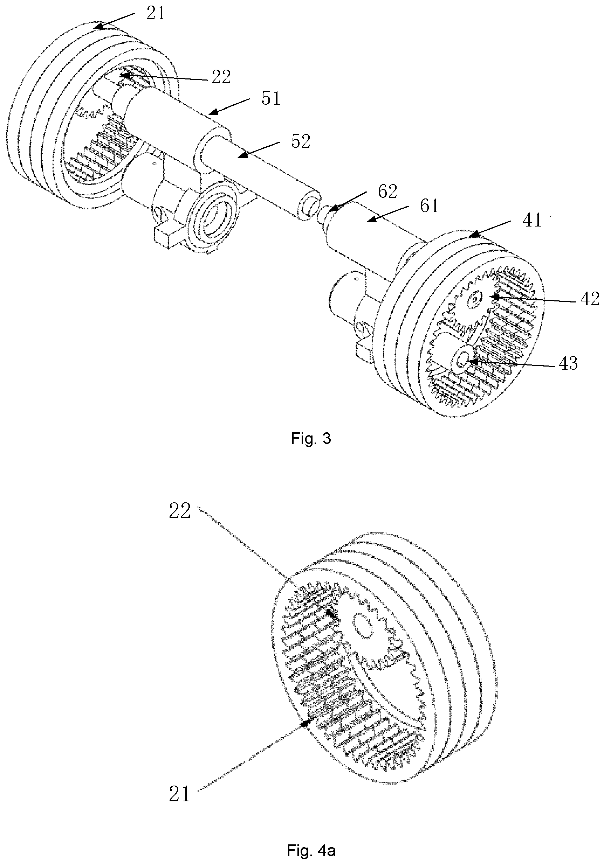

Fig. 1 is a schematic perspective diagram showing the structure of an implant delivery system according to an embodiment of the present invention. Figs. 2a to 2b are schematic partial views of the implant delivery system according to an embodiment of the present invention. Fig. 3 is a schematic partial view of the implant delivery handle according to an embodiment of the present invention. As shown in Figs. 1 to 3, in an embodiment of the present invention, there is provided an implant delivery system including a catheter assembly and an implant delivery handle. The implant delivery handle is configured to cause axial movement of first and second inner tubes.

The catheter assembly includes a first inner tube 11, a second inner tube 12 and a guidewire tube 13, which nest one within another from the outside inwards. That is, the first inner tube 11 nests the second inner tube 12, the second inner tube 12 nests on the guidewire tube 13. A guidewire is provided in the guidewire tube 13.

A distal end of the catheter assembly further includes a tapered head and a retaining head. The retaining head is provided at a distal end of the second inner tube 12 so as to be restricted in six degrees of freedom and thereby retain an implant 7. The tapered head is detachably provided at a distal end of the first inner tube 11, and the implant 7 is crimped on the distal end of the second inner tube 12 within the distal end of the first inner tube 11. Before being released, the implant 7 is confined between the tapered head and the retaining head. The implant 7 is a prosthetic heart valve, for example.

The implant delivery handle includes a housing 3, a first power transmission system, a second power transmission system, a first linkage assembly 5 and a second coupling member. The first and second power transmission systems are sequentially arranged from distal to proximal. A proximal end of the first power transmission system is inserted in the housing 3, while a distal end of the first power transmission system is exposed outside of a distal end of the housing 3. A distal end of the second power transmission system is inserted in the housing 3, while a proximal end of the second power transmission system is exposed outside of a proximal end of the housing 3. The first linkage assembly 5 is coupled to the distal end of the housing 3 and the first power transmission system, and the second coupling member is coupled to the proximal end of the housing 3 and the second power transmission system. The proximal end of the first inner tube 11 is secured to the first power transmission system, allowing the first power transmission system to drive axial movement of the first inner tube 11. Both the proximal end of the second inner tube 12 and the proximal end of the guidewire tube 13 are axially inserted through the first power transmission system and secured to the second power transmission system, allowing the second power transmission system to drive axial movement of the second inner tube 12.

The first power transmission system includes a first power transmission assembly 2 and the first linkage assembly 5, which are sequentially arranged from the distal end to the proximal end. The second power transmission system includes a second power transmission assembly 4 and a second linkage assembly 6, which are sequentially arranged from the proximal end to the distal end. The housing 3 is positioned between the first power transmission assembly 2 and the second power transmission assembly 4, and both the first linkage assembly 5 and the second linkage assembly 6 are provided in the housing 3. The first linkage assembly 5 is located at the distal end of the housing 3, while the second linkage assembly 6 is situated at the proximal end of the housing 3. A first coupling member is coupled to the housing 3 and the first power transmission system, and the second coupling member is coupled to the housing 3 and the second power transmission assembly 4.

The proximal end of the first inner tube 11 is inserted through the first power transmission assembly 2 and secured to the first linkage assembly 5, and the proximal end of the second inner tube 12 protrudes out of the proximal end of the first inner tube 11, passes through the first linkage assembly 5 and is then secured to the second linkage assembly 6. The proximal end of the guidewire tube 13 protrudes out of the proximal end of the second inner tube 12, passes through the second linkage assembly 6 and is then secured to the second power transmission assembly 4. When the first power transmission assembly 2 is rotating, the first linkage assembly 5 will drive the first inner tube 11 to move axially. When the second power transmission assembly 4 is rotating, the second linkage assembly 6 will drive the second inner tube 12 to move axially.

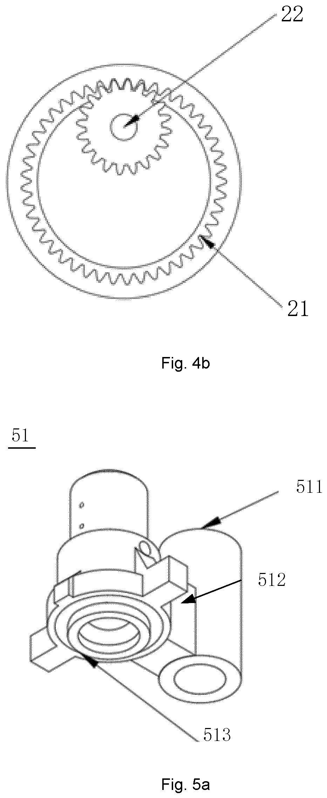

Figs. 4a to 4b are schematic diagrams showing the structure of the first power transmission assembly according to an embodiment of the present invention. As shown in Figs. 4a to 4b, with combined reference to Fig. 3, the first power transmission assembly 2 includes a first hand wheel, a first outer gear 21 and a first inner gear 22, which nest one within another from the outside inwards. The first hand wheel is secured over the first outer gear 21, and the first outer gear 21 and the first inner gear 22 make up a planetary gearset. The first outer gear 21 and the first inner gear 22 mesh with each other in a manner capable of transmitting power. When the first hand wheel is rotated, the first outer gear 21 and the first inner gear 22 will rotate in synchronization (e.g., counterclockwise or clockwise), driving the first inner tube 11 to axially move forth or back.

The first linkage assembly 5 includes a first inner tube coupling member 51 and a first lead screw 52. The first inner tube coupling member 51 is disposed over the first lead screw 52 in such a manner that it distally protrudes out of the first lead screw 52 and is then coupled to the first inner gear 22. The proximal end of the first inner tube 11 is inserted through the first outer gear 21 and secured to the distal end of the first inner tube coupling member 51. The proximal end of the second inner tube 12 protrudes out of the proximal end of the first inner tube 11, passes through the first inner tube coupling member 51 and is then secured to the second linkage assembly 6. Both the first inner tube 11 and the second inner tube 12 are arranged in parallel to the first lead screw 52. The first hand wheel can drive the first outer gear 21 and the first inner gear 22 to rotate in synchronization, and the first inner gear 22 can rotate coaxially the first lead screw 52, thereby driving the first inner tube 11 to move axially.

The first outer gear 21 is a cylindrical tubular structure which is open only at its proximal end, and the first inner gear 22 is disposed within the first outer gear 21 so as to be parallel to a bottom wall of the first outer gear 21. There are m grooves in an inner side wall surface of the first outer gear 21 and n teeth on an outer surface of the first inner gear 22, and m>n. The first outer gear 21 and the first inner gear 22 have equal modules and can therefore mesh with each other. Accordingly, the transmission ratio of the first outer gear 21 to the first inner gear 22, i.e., m: n, is greater than 1. For example, when the transmission ratio of the first outer gear 21 to the first inner gear 22 is 3, the first inner gear 22 makes three revolutions for every one revolution of the first outer gear 21. Similarly, the greater the m: n value, the greater the transmission ratio of the first outer gear 21 to the first inner gear 22. Higher power transmission efficiency of the first inner tube 11 can result in a reduction in the time required for a surgical procedure using the device and hence in the time over which the device stays in the patient's body, lowering the risk of accidents in the surgical procedure.

The first outer gear 21 has a first through hole (not shown) in its bottom wall, and the first inner tube 11 is inserted through the first through hole in the first outer gear 21. The first through hole has a diameter greater than an outer diameter of the first inner tube 11, allowing the axial movement of the first inner tube 11 through the first through hole.

The first inner tube coupling member 51 includes a first portion 511, a second portion 513 and a first fastener 512, which are arranged laterally side-by-side. The first fastener 512 is coupled to both the first portion 511 and the second portion 513.

The first portion 511 is a tubular structure, the first portion 511 is disposed over the first lead screw 52. Specifically, an internal thread is provided on an inner wall surface of the first portion 511, and an external thread complementary to the internal thread is provided on an outer surface of the first lead screw 52. The first portion 511 is screwed over the first lead screw 52 so as to drive axial movement of the second portion 513 as a result of rotation of the first lead screw 52.

The first fastener 512 defines two first guide posts on respective two opposing sides of the second portion 513, and the housing 3 has two axially-extending first guide channels. The first guide posts are received in the respective first guide channels. In this way, as a result of rotation of the first lead screw 52, the second portion 513 can move axially under the guidance of the first guide channels.

The second portion 513 defines a first lumen axially extending therethrough, and the proximal end of the first inner tube 11 is inserted through the first through hole and then secured at a distal end of the first lumen, allowing the first portion 511 to drive axial movement of the first inner tube 11. The proximal end of the second inner tube 12 protrudes out of the proximal end of the first inner tube 11, passes through the first lumen and is then secured to the second linkage assembly 6.

The second portion 513 includes a first seal 513a, a second seal 513b and a third seal 513c. A proximal end of the first seal 513a is secured over the third seal 513c, and the second seal 513b is sandwiched between the first seal 513a and the third seal 513c. The proximal end of the first inner tube 11 is secured within a distal end of the first seal 513a, and the second inner tube 12 extends out of the proximal end of the first inner tube 11 and passes through the first seal 513a, the second seal 513b and the third seal 513c. The second seal 513b seals a gap between the second inner tube 12 and the first lumen (i.e., between the second inner tube 12 and the first inner tube 11).

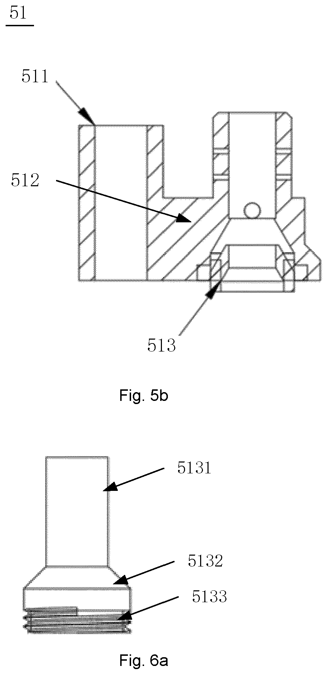

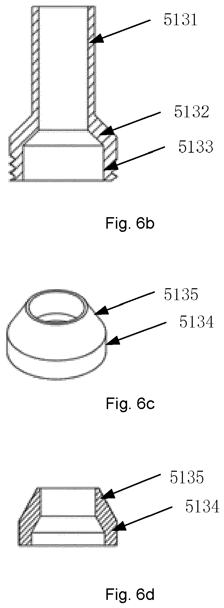

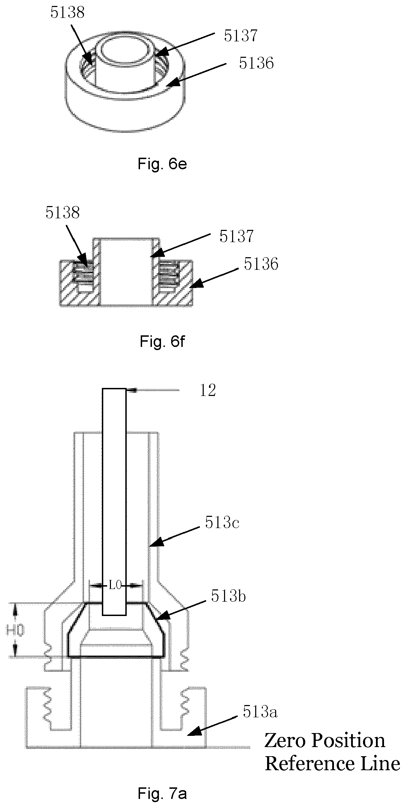

Figs. 6a to 6b are schematic diagrams showing the structure of the first seal according to an embodiment of the present invention. As shown in Figs. 6a to 6b, the first seal 513a is a tubular structure, the first seal 513a includes, joined together sequentially from distal to proximal, a first distal portion 5131, a first transition portion 5132 and a first proximal portion 5133. The first distal portion 5131 and the first proximal portion 5133 are both constant-diameter portions (i.e., the first distal portion 5131 has a constant outer diameter and a constant inner diameter, and the first proximal portion 5133 also has a constant outer diameter and a constant inner diameter), and the first transition portion 5132 is a varying-diameter portion. The first distal portion 5131 has the smallest outer diameter and the smallest inner diameter, and the inner diameter of the first distal portion 5131 is greater than the outer diameter of the first inner tube 11, allowing the proximal end of the first inner tube 11 to be secured to an inner wall surface of the first distal portion 5131 at the distal end thereof. The first proximal portion 5133 has the greatest outer diameter and the greatest inner diameter, and outer and inner diameters of the first transition portion 5132 both decrease from proximal to distal. An external thread is provided on an outer circumferential surface of the first proximal portion 5133 around a proximal end thereof.

Figs. 6c to 6d are schematic diagrams showing the structure of the second seal according to an embodiment of the present invention. As shown in Figs. 6c to 6d, the second seal 513b is generally formed of a flexible material (i.e., an elastic material), in particular a polymeric material such as silicone, thermoplastic polyurethane (TPU) or Pebax, and the second seal 513b generally has a Shore A hardness of 35 HA to 55 HA. The second seal 513b includes, joined together from proximal to distal, a second proximal portion 5134 and a second distal portion 5135. The second proximal portion 5134 is a constant-diameter portion, and the second distal portion 5135 is a varying-diameter portion. The distal end of the second distal portion 5135 has a minimum inner diameter and a minimum outer diameter. The outer diameter of the distal end of the second distal portion 5135 is greater than or equal to the inner diameter of the first distal portion 5131 and smaller than the inner diameter of the first proximal portion 5133, thus allowing the second seal 513b to be received in the first proximal portion 5133 and the first transition portion 5132, with the second proximal portion 5134 being located more proximally.

The second seal 513b defines a first interior space extending axially through the second seal 513b and including a first segment, a second segment and a third segment, which are sequentially joined together from proximal to distal. The first, second and third segments all have circular radial cross-sections. The first and third segments are both constant-diameter segments, while the second segment is a varying-diameter segment. The first segment has the greatest diameter, and the third segment has the smallest diameter, which is, however, greater than an outer diameter of the second inner tube, thereby allowing the second inner tube to be inserted in the second seal 513b. The second segment has a diameter gradually decreasing from proximal to distal.

Figs. 6e to 6f are schematic diagrams showing the structure of the third seal according to an embodiment of the present invention. As shown in Figs. 6e to 6f, the third seal 513c includes, joined together sequentially from proximal to distal, a third proximal portion 5136 and a third distal portion 5137, the third proximal portion 5136 and the third distal portion 5137 are both constant-diameter portions. The third proximal portion 5136 has a greater outer diameter than the third distal portion 5137, and the third proximal portion 5136 has the same inner diameter as the third distal portion 5137. The inner diameter of the third seal 513c is greater than the outer diameter of the second inner tube, allowing the second inner tube to be inserted in the third seal 513c. The distal end face of the third proximal portion 5136 defines an axially-extending annular first trough 5138. The first trough 5138 is delimited by a bottom wall surface at its proximal end, an inner side wall surface closer to its axis and an outer side wall surface farther away from its axis. The inner side wall surface is spaced from the axis by a distance equal to an outer diameter of the third distal portion 5137, and an internal thread complementary to the external thread on the outer circumferential surface of the first proximal portion 5133 is provided on the outer side wall surface. Accordingly, the first proximal portion 5133 can be screwed into the first trough 5138 so that the third distal portion 5137 is received within the first proximal portion 5133. During screwing of the first seal 513a into the first trough 5138, the third seal 513c will push the second seal 513b from its proximal side, thereby sandwiching the second seal 513b between the first seal 513a and the third seal 513c.

The outer diameter of the third distal portion 5137 is greater than the inner diameter of the second distal portion 5135 and smaller than the inner diameter of the first proximal portion 5133. As such, the first seal 513a and the third seal 513c can sandwich the second seal 513b therebetween in such a manner that the second seal 513b radially elastically deforms and the inner diameter of the second distal portion 5135 in the second seal 513b decreases until it elastically squeezes onto the second inner tube 12 and thereby seals the gap between the first inner tube 11 and the second inner tube 12, preventing the occurrence of air/liquid leakage or other undesirable conditions and resulting in a great increase in surgical safety.

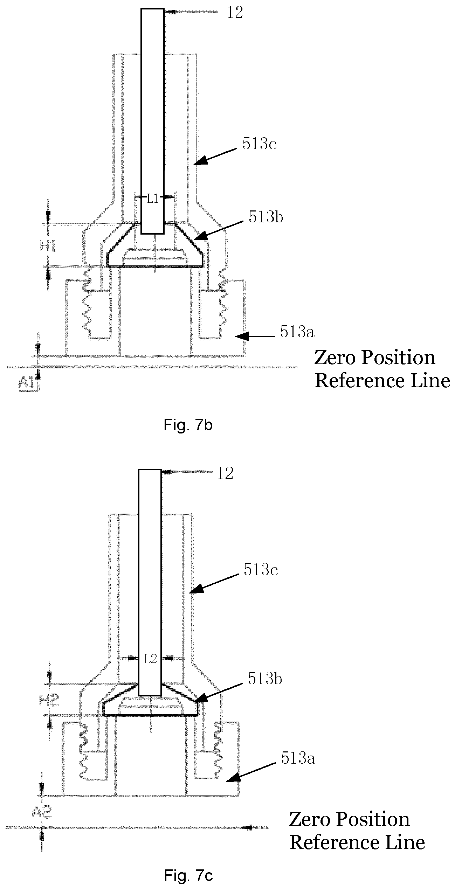

How the second portion 513 achieves sealing is explained in more detail now. Here, for clarity of description, a line on which a distal end face of the third seal 513c is located in an initial configuration of the first seal 513a, the second seal 513b and the third seal 513c is defined as a zero position reference line. As shown in Fig. 7a, at first, the first seal 513a, the second seal 513b and the third seal 513c are in the initial configuration, in which the inner diameter of the second distal portion 5135 is L0 that is greater than the outer diameter of the second inner tube 12. Moreover, the second seal 513b has an axial length of H0, and an axial direction between the third seal 513c and the zero position reference line is 0. Next, as shown in Fig. 7b, with the first seal 513a being held stationary, the third seal 513c is preliminarily tightened. As a result, the third seal 513c axially moves upward and the third seal 513c pushes the second seal 513b, causing elastic deformation of the second seal 513b. Accordingly, the inner diameter of the second distal portion 5135 decreases to L1 that is still greater than the outer diameter of the second inner tube 12. Meanwhile, the axial length of the second seal 513b decreases to H1, and the axial distance between the third seal 513c and the zero position reference line increases to A1. At last, as shown in Fig. 7c, with the first seal 513a still being stationary, the third seal 513c is further tightened. As a result, it further axially moves upward and additionally pushes the second seal 513b, increasing the elastic deformation of the second seal 513b and causing it to squeeze onto an outer wall surface of the second inner tube 12. Accordingly, the inner diameter of the second distal portion 5135 further decreases to L2 that is equal to the outer diameter of the second inner tube 12. Moreover, the axial length of the second seal 513b further decreases to H2, and the axial distance between the third seal 513c and the zero position reference line further increases to A2. As such, the second inner tube 12 is sealed with the first lumen. In this embodiment, the hardness of the second seal 513b, the inner diameter of the second transition portion, the axial length of the second seal 513b and the axial distance between the third seal 513c and the zero position reference line may be designed according to the outer diameter of the second inner tube.

As shown in Fig. 3, the second power transmission assembly 4 includes a second hand wheel, a second outer gear 41 and a second inner gear 42, nest one within another from the outside inwards. The second hand wheel is secured over the second outer gear 41, and the second outer gear 41 and the second inner gear 42 make up a planetary gearset. The second outer gear 41 and the second inner gear 42 mesh with each other in a manner capable of transmitting power. The second outer gear 41 and the second inner gear 42 rotate in synchronization (e.g., counterclockwise or clockwise), driving the second inner tube 12 to axially move forth or back.

As shown in Figs. 2b and 3, the second linkage assembly 6 includes a second inner tube coupling member 61 and a second lead screw 62. The second inner tube coupling member 61 is disposed over the second lead screw 62, the proximal end of the second inner tube coupling member 61 protrudes out of the second lead screw 62 and is then coupled to the second inner gear 42. The proximal end of the second inner tube 12 is inserted through the first linkage assembly 5 and secured to the second inner tube coupling member 61, the second inner tube 12 extends in parallel to the second lead screw 62. The proximal end of the guidewire tube 13 protrudes out of the proximal end of the second inner tube 12, passes through the second inner tube coupling member 61 and is then secured to the second outer gear 41. The second hand wheel can drive the second outer gear 41 and the second inner gear 42 to rotate in synchronization, and the second inner gear 42 can rotate coaxially the second lead screw 62, thereby driving the second inner tube 12 to move axially.

The second outer gear 41 is a cylindrical tubular structure which is open only at its proximal end, and the second inner gear 42 is disposed within the second outer gear 41 so as to be parallel to a bottom wall of the second outer gear 41. The second lead screw 62 is passed through the bottom wall of the second outer gear 41 and then coupled to the second inner gear 42 within the second outer gear 41, allowing the second inner gear 42 to drive rotation of the second lead screw 62.

There are M grooves in an inner side wall surface of the second outer gear 41 and N teeth on an outer surface of the second inner gear 42, and M>N. The second outer gear 41 and the second inner gear 42 have equal modules and can therefore mesh with each other. Accordingly, the transmission ratio of the second outer gear 41 to the second inner gear 42, i.e., M: N, is greater than 1. The greater the M: N value, the greater the transmission ratio of the second outer gear 41 to the second inner gear 42. Higher power transmission efficiency of the second inner tube 12 can result in a reduction in the time required for a surgical procedure using the device and hence in the time over which the device stays in the patient's body, lowering the risk of accidents in the surgical procedure. M may be equal to m or not, and N may be equal to n or not, depending on the requirements of practical applications.

Referring to Figs. 1 and 3, the bottom wall of the second outer gear 41 is attached with a guidewire tube fastener 43, the guidewire tube fastener 43 is axially inserted through the bottom wall of the second outer gear 41 and fixes the proximal end of the guidewire tube 13. The proximal end of the second outer gear 41 is provided with a gear cover, the gear cover closes the opening of the second outer gear 41 to provide protection to the component within the second outer gear 41.

The second inner tube coupling member 61 includes a third portion, a fourth portion and a second fastener (not shown), which are arranged laterally side-by-side. The second fastener is coupled to and between the third portion and the fourth portion.

The third portion is a tubular structure, the third portion is disposed over the second lead screw 62. Specifically, an internal thread is provided on an inner wall surface of the third portion, and an external thread complementary to the internal thread is provided on an outer surface of the second lead screw 62. The third portion is screwed over the second lead screw 62, the third portion drives axial movement of the fourth portion as a result of rotation of the second lead screw 62.

The second fastener defines two second guide posts on respective two opposing sides of the fourth portion, and the housing 3 has two axially-extending second guide channels. The second guide posts are received in the respective second guide channels. In this way, as a result of rotation of the second lead screw 62, the fourth portion can move axially under the guidance of the second guide channels.

The fourth portion defines a second lumen axially extending therethrough, and the proximal end of the second inner tube 12 is inserted through the first lumen and then secured at a distal end of the second lumen, allowing the third portion to drive axial movement of the second inner tube 12. The proximal end of the guidewire tube 13 protrudes out of the proximal end of the second inner tube 12, passes through the second lumen and is then secured to the guidewire tube fastener 43.

The fourth portion includes a fourth seal, a fifth seal and a sixth seal. A proximal end of the fourth seal is secured over the sixth seal, and the fifth seal is sandwiched between the fourth and sixth seals. The proximal end of the second inner tube 12 is secured within a distal end of the fourth seal, and the proximal end of the guidewire tube 13 protrudes out of the proximal end of the second inner tube 12, passes through the fourth, fifth and sixth seals and is then secured to the guidewire tube fastener 43. The fifth seal seals a gap between the guidewire tube 13 and the second lumen (i.e., between the guidewire tube 13 and the second inner tube 12).

The fourth seal is a tubular structure, the fourth seal includes, joined together sequentially from distal to proximal, a first distal portion, a first transition portion and a first proximal portion. The first distal portion and the first proximal portion are both constant-diameter portions (i.e., the first distal portion has a constant outer diameter and a constant inner diameter, and the first proximal portion also has a constant outer diameter and a constant inner diameter), and the first transition portion is a varying-diameter portion. The first distal portion has the smallest outer diameter and the smallest inner diameter, and the inner diameter of the first distal portion is greater than an outer diameter of the guidewire tube 13. The first proximal portion has the greatest outer diameter and the greatest inner diameter, and outer and inner diameters of the first transition portion both decrease from proximal to distal. An external thread is provided on an outer circumferential surface of the first proximal portion around a proximal end thereof.

The fifth seal is generally formed of a flexible material, in particular a polymeric material such as silicone, TPU or Pebax, and generally has a Shore A hardness of 35 HA to 55 HA. The fifth seal includes, joined together from proximal to distal, a second proximal portion and a second distal portion. The second proximal portion is a constant-diameter portion, and the second distal portion is a varying-diameter portion. The second distal portion has a minimum inner diameter and a minimum outer diameter at its distal end. The outer diameter of the second distal portion at its distal end is greater than or equal to the inner diameter of the first distal portion and smaller than the inner diameter of the first proximal portion, thus allowing the fifth seal to be received in the first proximal portion and the first transition portion, with the second proximal portion being located more proximally.

The fifth seal defines a second interior space extending axially through the fifth seal and including a first segment, a second segment and a third segment, which are sequentially joined together from proximal to distal. The first, second and third segments of the second interior space all have circular radial cross-sections. The first and third segments of the second interior space are both constant-diameter segments, while the second segment of the second interior space is a varying-diameter segment. The first segment of the second interior space has the greatest diameter, and the third segment of the second interior space has the smallest diameter, which is, however, greater than the outer diameter of the guidewire tube. The second segment of the second interior space has a diameter gradually decreasing from proximal to distal.

The sixth seal includes, joined together sequentially from proximal to distal, a third proximal portion and a third distal portion, the third proximal portion and the third distal portion of the sixth seal are both constant-diameter portions. The third proximal portion of the sixth seal has a greater outer diameter than the third distal portion of the sixth seal and the same inner diameter as the third distal portion of the sixth seal. The inner diameter of the sixth seal is greater than the outer diameter of the guidewire tube. The distal end face of the third proximal portion of the sixth seal defines an axially-extending annular second trough. The second trough is delimited by a bottom wall surface at its proximal end, an inner side wall surface closer to its axis and an outer side wall surface farther away from its axis. The inner side wall surface of the second trough is spaced from the axis by a distance equal to an outer diameter of the third distal portion of the sixth seal, and an internal thread complementary to the external thread on the outer circumferential surface of the first proximal portion of the fourth seal is provided on the outer side wall surface of the second trough. Accordingly, the first proximal portion of the fourth seal can be screwed into the second trough so that the third distal portion of the sixth seal is received within the first proximal portion of the fourth seal. During screwing of the fourth seal into the second trough, the sixth seal will push the fifth seal from its proximal side, thereby sandwiching the fifth seal between the fourth and sixth seals, preventing the occurrence of air/liquid leakage or other undesirable conditions and resulting in a great increase in surgical safety.

The outer diameter of the third distal portion of the sixth seal is greater than the inner diameter of the second distal portion of the fifth seal and smaller than the inner diameter of the first proximal portion of the fourth seal. As such, the fourth and sixth seals can sandwich the fifth seal therebetween in such a manner that the fifth seal radially elastically deforms and the inner diameter of the second distal portion in the fifth seal decreases until it elastically squeezes onto the guidewire tube 13 and thereby seals the gap between the guidewire tube 13 and the second inner tube 12. The fourth portion achieves sealing in the same manner as the second portion does.

The implant delivery handle of the present invention is assembled by inserting the proximal end of the first inner tube 11 through the first through hole and securing it at the distal end of the first seal 513a in the first inner tube coupling member 51. The proximal end of the second inner tube is then inserted through the first inner tube and then the first lumen and secured at the distal end of the fourth seal in the second inner tube coupling member 61. The third seal 513c is tightened against the first seal 513a to compress the second seal 513b therebetween, causing the second seal 513b to deform and squeeze on the outer wall surface of the second inner tube and thereby seal the gap between the outer wall surface of the proximal end of the second inner tube 12 and the first inner tube 11. The guidewire tube 13 is inserted through the second inner tube 12 until it protrudes out of its proximal end and passes through the second lumen. After that, it is secured to the guidewire tube fastener 43 of the second outer gear 41. The sixth seal tightened against the fourth seal to compress the fifth seal therebetween, causing the fifth seal to deform and squeeze on the outer wall surface of the guidewire tube 13 and thereby seal the gap between the outer wall surface of the proximal end of the guidewire tube 13 and the second inner tube 12.

The distal end of the first lead screw 52 is inserted through the first portion 511 of the first inner tube coupling member 51 and then engaged with the first inner gear 22. As such, as a result of the first hand wheel being turned, the first inner gear 22 and the first outer gear 21 rotate simultaneously, causing rotation of the first lead screw 52 threadedly meshed with the first inner gear 22 and hence axial movement of the first inner tube 11 secured to the second portion 513 of the first inner tube coupling member 51. The proximal end of the second lead screw 62 is inserted through the third portion of the second inner tube coupling member 61 and then engaged with the second inner gear 42. As such, as a result of the second hand wheel being turned, the second inner gear 42 and the second outer gear 41 rotate simultaneously, causing rotation of the second lead screw 62 threadedly meshed with the second inner gear 42 and hence axial movement of the second inner tube 12 secured to the fourth portion of the second inner tube coupling member 61. Since the first inner gear 22 and the first outer gear 21 make up a planetary gearset and the second inner gear 42 and the second outer gear 41 make up another planetary gearset, large load capacity and a high transmission ratio can be provided to enable the handle to address various applications, in particular those requiring a high transmission ratio (i.e., high power transmission efficiency).

Referring to Figs. 1 to 3, in embodiments of the present invention, there is provided a method of use of the above-discussed implant delivery system, for example, for release of an implant 7 for two-step release. The method includes: providing the implant delivery system by assembling the implant delivery handle with the catheter assembly in the manner described above; then rotating both the first and second hand wheels in the first direction to drive the power transmission assembly consisting of the first outer gear 21, the first inner gear 22 and the first lead screw 52 to axially retract the first inner tube 11 and to drive the power transmission assembly consisting of the second outer gear 41, the second inner gear 42 and the second lead screw 62 to simultaneously axially retract the second inner tube 12; and subsequently, rotating both the first and second hand wheels in the direction opposite to the first direction to drive the power transmission assembly consisting of the first outer gear 21, the first inner gear 22 and the first lead screw 52 to axially advance the first inner tube 11 and to drive the power transmission assembly consisting of the second outer gear 41, the second inner gear 42 and the second lead screw 62 to simultaneously axially advance the second inner tube 12, thereby loading the implant 7 into a delivery tube; afterwards, advancing the implant delivery system to a target site along a predetermined path; and then rotating both the first and second hand wheels in the first or opposite direction to axially move the first inner tube 11 and the second inner tube 12, thereby completing release of the implant 7.

In summary, in the implant delivery handle, the implant system, the delivery system and the method of the present invention, the planetary gearsets are used in combination with the lead screws to provide power transmission. This enables the implant delivery handle to have large load capacity and a high transmission ratio, which result in faster power transmission. As a result, the time required for a surgical procedure using the device and hence the time over which the device stays in the patient's body, as well as the risk of accidents in the surgical procedure, can be reduced. As for delivery catheter sealing, compared with conventional designs, the seal design adopted by the first and second inner tube coupling members of the present invention is structurally simpler and safer. This simple structure can result in remarkable cost savings in terms of both design and fabrication.

It is to be noted that, as used herein, the terms "first" and "second" are only meant to distinguish various components, elements, steps, etc. from each other rather than indicate logical or sequential orderings thereof, unless otherwise indicated or specified.

It is to be understood that while the invention has been described above with reference to preferred embodiments thereof, it is not limited to these embodiments. In light of the above teachings, any person familiar with the art may make many possible modifications and variations to the disclosed embodiments or adapt them into equivalent embodiments, without departing from the scope of the invention. Accordingly, it is intended that any and all simple variations, equivalent changes and modifications made to the foregoing embodiments based on the substantive disclosure of the invention without departing from the scope thereof fall within this scope.

## Claims

1. An implant delivery handle, comprising two transmission systems arranged along an axial direction of a first inner tube, a distal one of the transmission systems coupled to a proximal end of the first inner tube, a proximal one of the transmission systems coupled to a proximal end of a second inner tube, and
wherein each of the transmission systems comprises a lead screw, an inner tube coupling member, an outer gear and an inner gear, the outer gear rotatably engaging the inner gear and configured to drive the inner gear to rotate, the lead screw connected to the first inner tube or the second inner tube through the inner tube coupling member, the lead screw also engaging the inner gear and rotating with the inner gear, thereby driving the first inner tube or the second inner tube to axially move, wherein a transmission ratio of the outer gear to the inner gear is greater than 1.

2. The implant delivery handle of claim 1, wherein the outer gear is disposed over the inner gear, and the outer gear meshes with the inner gear.

3. The implant delivery handle of claim 1, wherein:
the inner tube coupling member in the distal transmission system is connected to an outer wall surface of the proximal end of the first inner tube, disposed over the second inner tube and configured to proximally seal a gap between the first inner tube and the second inner tube, and
the inner tube coupling member in the proximal transmission system is connected to an outer wall surface of the proximal end of the second inner tube, disposed over a guidewire tube in a catheter assembly and configured to proximally seal a gap between the second inner tube and the guidewire tube.

4. The implant delivery handle of claim 1, wherein:
the inner tube coupling member in the distal transmission system comprises a first seal, a second seal and a third seal, which are disposed over the second inner tube, wherein the proximal end of the first inner tube is snugly secured in a distal end of the first seal, and the second seal is elastically switched between a proximal end of the first seal and a distal end of the third seal, when the second seal is compressed by both the first and third seals, the second seal fits against the outer wall surface of the second inner tube, thereby sealing the gap between the proximal end of the first inner tube and the second inner tube; and
the inner tube coupling member in the proximal transmission system comprises a fourth seal, a fifth seal and a sixth seal, which are disposed over the guidewire tube in the catheter assembly, wherein the proximal end of the second inner tube is snugly secured in a distal end of the fourth seal, and the fifth seal is elastically switched between a proximal end of the fourth seal and a distal end of the sixth seal, when the fifth seal is compressed by both the fourth and sixth seals, the fifth seal fits against an outer wall surface of the guidewire tube, thereby sealing the gap between the proximal end of the second inner tube and the guidewire tube.

5. The implant delivery handle of claim 4, wherein the second and fifth seals are each made of a material comprising a polymeric material and the second and fifth seals each have a Shore A hardness of 35 HA to 55 HA.

6. The implant delivery handle of claim 4, wherein:
the first, second and third seals are all tubular structures, the second seal is disposed within the proximal end of the first seal, and the distal end of the third seal is disposed within the proximal end of the first seal and configured to act together with the first seal to compress the second seal so that the second seal experiences an inner diameter decrease and fits against the second inner tube,
inner diameters of the first, second and third seals are all greater than or equal to an outer diameter of the second inner tube, an inner diameter of the proximal end of the first seal is greater than an inner diameter of the distal end of the first seal, an outer diameter of the second seal lies between the inner diameter of the distal end of the first seal and the inner diameter of the proximal end of the first seal, and an outer diameter of the distal end of the third seal lies between an inner diameter of a distal end of the second seal and the inner diameter of the proximal end of the first seal.

7. The implant delivery handle of claim 4, wherein:
the fourth, fifth and sixth seals are all tubular structures, the fifth seal is disposed within the proximal end of the fourth seal, and the distal end of the sixth seal is disposed within the proximal end of the fourth seal and configured to act together with the fourth seal to compress the fifth seal so that the fifth seal experiences an inner diameter decrease and fits against the guidewire tube,
inner diameters of the fourth, fifth and sixth seals are all greater than or equal to an outer diameter of the guidewire tube, an inner diameter of the proximal end of the fourth seal is greater than an inner diameter of the distal end of the fourth seal, an outer diameter of the fifth seal lies between the inner diameter of the distal end of the fourth seal and the inner diameter of the proximal end of the fourth seal, and an outer diameter of the distal end of the sixth seal lies between an inner diameter of a distal end of the fifth seal and the inner diameter of the proximal end of the fourth seal.

8. The implant delivery handle of claim 1, wherein: the lead screw is arranged coaxially with the inner gear, the lead screw has an external thread, the inner tube coupling member has an internal thread, the inner tube coupling member threadedly engages with the lead screw, and as a result of threaded rotation of the lead screw, the inner tube coupling member drives axial movement of the first inner tube or the second inner tube.

9. An implant delivery system, comprising the implant delivery handle of any one of claims 1 to 8 and a catheter assembly, the catheter assembly comprising the first inner tube, the second inner tube and a guidewire tube, which nest one within another from the outside inwards, wherein the implant delivery handle drives axial movement of the first and second inner tubes.

10. An implant system, comprising the implant delivery handle of any one of claims 1 to 8, the first inner tube, the second inner tube, a guidewire tube, a tapered head and a retaining head, the retaining head disposed at distal ends of the second inner tube and the guidewire tube, the tapered head disposed at a distal end of the first inner tube, wherein an implant is crimped within a distal end portion of the first inner tube between the tapered head and the retaining head, and wherein the implant delivery handle drives axial movement of the first and second inner tubes, thereby achieving loading, delivery and release of the implant.

11. A method of use of the implant delivery system of claim 9, the method comprising:
rotating the outer gear in a first direction, causing simultaneous rotation of the inner gear with the outer gear, and the lead screw rotating with the inner gear, the rotation of the lead screw causes the inner tube coupling member to drive axial advancement of the first inner tube or the second inner tube; and
rotating the outer gear in a direction opposite to the first direction, causing synchronous rotation of the inner gear with the outer gear, and the lead screw rotating with the inner gear, the rotation of the lead screw causes the inner tube coupling member to drive axially retraction of the first inner tube or the second inner tube.
